(19) **Europäisches Patentamt / European Patent Office / Office européen des brevets**

(11) **EP 3 811 744 B1**

(12) **FASCICULE DE BREVET EUROPEEN**

(45) Date de publication et mention de la délivrance du brevet:
**10.12.2025  Bulletin 2025/50**

(21) Numéro de dépôt: **19759660.4**

(22) Date de dépôt: **29.07.2019**

(51) Classification Internationale des Brevets (IPC):
**H05K 1/18** *(2006.01)*    **H05K 1/02** *(2006.01)*
**H05K 3/40** *(2006.01)*    **H05K 3/00** *(2006.01)*
**H01L 21/56** *(2006.01)*    **H01L 21/683** *(2006.01)*
**H01L 23/14** *(2006.01)*    **H01L 23/538** *(2006.01)*
**H01L 23/00** *(2006.01)*

(52) Classification Coopérative des Brevets (CPC):
**H05K 1/189; H01L 21/561; H01L 21/6835;
H01L 23/145; H01L 23/5384; H01L 23/5387;
H01L 24/97; H05K 1/0281; H05K 3/4007;**
H01L 21/568; H01L 24/13; H01L 24/16; H01L 24/81;
H01L 2221/68327; H01L 2221/68331;        (Cont.)

(86) Numéro de dépôt international:
**PCT/FR2019/051865**

(87) Numéro de publication internationale:
**WO 2020/025889 (06.02.2020 Gazette 2020/06)**

(54) **STRUCTURE ELECTRONIQUE SOUPLE ET SON PROCEDE D'ELABORATION**

FLEXIBLE ELEKTRONISCHE STRUKTUR UND HERSTELLUNGSVERFAHREN DAFÜR

FLEXIBLE ELECTRONIQUE STRUCTURE AND MANUFACTURING PROCESS THEREFORE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **30.07.2018  FR 1857094**

(43) Date de publication de la demande:
**28.04.2021   Bulletin 2021/17**

(73) Titulaire: **Commissariat à l'Energie Atomique et aux Energies Alternatives
75015 Paris (FR)**

(72) Inventeurs:
• **SOURIAU, Jean-Charles
38054 GRENOBLE CEDEX 09 (FR)**
• **BAILLIN, Xavier
38054 GRENOBLE CEDEX 09 (FR)**

(74) Mandataire: **Santarelli
Tour Trinity
1 bis Place de la Défense
92400 Courbevoie (FR)**

(56) Documents cités:
WO-A1-2013/082537      US-A1- 2006 207 088
US-A1- 2009 002 973     US-A1- 2009 107 703
US-A1- 2015 157 862     US-A1- 2018 027 651
US-A1- 2018 042 107     US-A9- 2017 186 533
US-B2- 10 187 975

**(Cont. page suivante)**

(52) Classification Coopérative des Brevets (CPC):
(Cont.)H01L 2221/6834; H01L 2221/68345;
H01L 2221/68381; H01L 2224/16225;
H01L 2224/16227; H01L 2224/16245;
H01L 2224/81001; H01L 2224/81005;
H01L 2224/81203; H01L 2224/81801;

H01L 2224/8185; H01L 2224/97; H01L 2924/15159;
H05K 1/0271; H05K 1/183; H05K 3/0052;
H05K 3/007; H05K 2201/10803; H05K 2201/2009;
H05K 2203/016; H05K 2203/0384;
H05K 2203/1184; H05K 2203/308

**Description**

**DOMAINE TECHNIQUE ET ÉTAT DE LA TECHNIQUE ANTÉRIEURE**

La présente invention se rapporte à une structure électronique souple, intégrant un ou plusieurs composants électroniques, capable de se déformer et pouvant être posée, sur une surface non plane telle que la peau ou un objet.

La présente invention se rapporte également au procédé de préparation d'une telle structure.

Les structures électroniques souples, aussi appelées dispositifs électroniques souples, peuvent intégrer des composants électroniques tels que des moyens de communications (antenne), des circuits intégrés, des actuateurs, des batteries, des puces RFID ou encore des composants passifs.

Pour réaliser de telles structures, il est possible de reporter des composants électroniques sur un circuit imprimé flexible de type polyester, polyimide, polytétrafluoroéthylène, polyétheréthercétone. Les circuits imprimés comprennent des pistes métalliques et des plots d'interconnexion sur lesquels les composants sont fixés par brasure, à l'aide de matériaux fusibles ou encore par collage par exemple. Les composants sont fixés en surface du film souple, soit avec la face active vers le bas soit avec la face active vers le haut, et interconnectés à l'aide de fils électriquement conducteurs (« wire bonding »). Généralement, les pistes métalliques sont recouvertes d'un vernis épargne ou d'un film diélectrique qui sert de masque anti-soudure et de couche isolante et protectrice contre la corrosion et l'endommagement.

Cependant, comme les composants électroniques sont souvent épais et rigides, les contraintes en flexion dans la structure sollicitent fortement les interfaces de collage, de soudure ou de brasure, qui finissent par rompre.

Pour remédier à ce problème, une solution consiste, à utiliser des composants électroniques de plus faibles épaisseurs, pour qu'ils soient plus souples. Par exemple, dans le document US-A-2007/0134849, il est proposé un dispositif (« Ultra-Thin Package » (UTCP)) dans lequel une puce a une épaisseur de 10 μm à 50 μm. Pour réaliser le dispositif, la puce, après avoir été amincie, est fixée sur un substrat rigide, face active vers le haut (à l'opposé du substrat). Le substrat peut être du verre recouvert d'une couche de polyimide de 20 μm d'épaisseur. La puce est, par exemple, fixée sur le substrat grâce à une couche de bicyclobutane benzocyclobutène (BCB) ou de polyimide. Une fois fixée, la puce est recouverte par une autre couche de polyimide de 20 μm d'épaisseur. La couche supérieure de polyimide est ensuite gravée pour pouvoir réaliser la prise de contact à travers la couche supérieure et les pistes métalliques sont réalisées en surface de la structure. La structure souple est finalement séparée du substrat rigide. Par exemple, pour des puces de 20-30 μm d'épaisseur,

encapsulée entre deux couches de polyimide de 20 μm d'épaisseur, la structure finale souple a une épaisseur de 60-70 μm.

Cependant, cette structure est réalisable uniquement avec des puces ultra-minces (ici d'épaisseurs inférieures à 50 μm). Or, les composants disponibles sur le marché ont rarement de si faibles épaisseurs, et il n'est pas envisageable, d'un point de vue industriel, d'amincir individuellement chaque composant électronique pour pouvoir le reporter ensuite dans une structure souple. De plus, les pistes métalliques et les prises de contact sont soumises aux contraintes de flexion et de torsion, ce qui peut les fragiliser et ainsi réduire la durée de vie de la structure.

Dans le document US-A-2006/0097373, il est proposé un dispositif électronique dans lequel une puce semi-conductrice ultra-fine est collée sur un substrat souple. La puce semi-conductrice intègre un plot de connexion. Ce dernier affleure à la surface supérieure ou inférieure de la puce, au niveau du plan neutre du dispositif électronique. Cet agencement permet de limiter des contraintes au niveau de la connexion électrique de la puce semi-conductrice, lorsque le dispositif est plié.

Le document US 2018/042107 divulgue un substrat multicouche comportant un corps de substrat défini par un stratifié de matériaux isolants flexibles, un composant de faible épaisseur et des composants d'épaisseur importante. Les matériaux du corps de substrat sont empilés et peuvent être principalement constitués d'un cristal liquide polymère.

Le document WO 2013/082537 divulgue un dispositif d'éclairage flexible à semi-conducteurs comprenant un substrat porteur ayant des éléments d'alignement sur un premier côté, des éléments émetteurs de lumière couplés de façon fonctionnelle aux éléments d'alignement, des éléments électriquement conducteurs couplés de façon fonctionnelle au premier côté.

Un objectif de la présente invention est de proposer une structure souple ayant une durée de vie encore améliorée par rapport à l'art antérieur, et notamment une meilleure résistance aux contraintes mécaniques en flexion.

Un autre objectif de la présente invention est de proposer un procédé pour réaliser une telle structure, qui soit facile à mettre en œuvre, qui ne nécessite pas d'amincir individuellement chaque composant avant sa mise en place dans la structure, et qui puisse être transposé à une échelle industrielle.

**EXPOSÉ DE L'INVENTION**

L'invention est divulguée par les revendications jointes.

Ces objectifs sont atteints avec une structure électronique souple comprenant :

un premier film, en un premier polymère ou en verre,

un deuxième film, en un deuxième polymère, dans lequel est disposé au moins un composant électronique, le deuxième film recouvrant le premier film, au moins une piste électriquement conductrice disposée entre le premier film et le deuxième film, et électriquement connectées chacune à l'au moins un composant électronique, par un élément d'interconnexion respectif,

éventuellement un troisième film en un troisième polymère ou en verre, recouvrant le deuxième film.

Chaque élément d'interconnexion est disposé à proximité du plan neutre de la structure, c'est-à-dire passant par un plan dont la distance au plan neutre est inférieure ou égale à 20% de l'épaisseur totale de la structure.

Selon l'invention, la structure électronique souple comprend en outre au moins une couche de compensation discontinue, formée d'une ou plusieurs portions discrètes qui s'étendent chacune en vis-à-vis de l'une piste électriquement conductrice ou en vis-à-vis de l'un composant électronique.

La couche de compensation peut être formée distincte des premier, deuxième et troisième films, constituée d'une ou plusieurs portions discrètes en un matériau distinct du matériau respectif formant chacun desdits films.

En variante, la couche de compensation peut être en verre et formée d'un seul tenant avec le troisième film lorsqu'il est en verre. En d'autres termes, la couche de compensation forme alors une ou plusieurs surépaisseurs locales dans une couche en verre formant à la fois le troisième film et la couche de compensation. Dit encore autrement, une couche en verre formant à la fois le troisième film et la couche de compensation présente une topographie de surface, du côté opposé au deuxième film, avec un ou plusieurs plateaux qui forment la couche de compensation.

Par souple, on entend que la structure est flexible, c'est-à-dire qu'elle peut subir une flexion d'un rayon de courbure inférieur ou égal à 1000 mm et de préférence inférieur ou égal à 200 mm, sans rupture. La présence des deux ou trois films en polymère ou en verre assure la souplesse du dispositif. En particulier, le deuxième film présente avantageusement un module de Young inférieur ou égal à 5000MPa, et de préférence, inférieur ou égal à 3500MPa. De la même façon, le premier film et le troisième film, lorsqu'ils sont en un polymère, présentent chacun un module de Young inférieur ou égal à 5000MPa, et de préférence, inférieur ou égal à 3500MPa. Lorsque le premier, respectivement le troisième film est en verre, il présente alors un module de Young supérieur à 50 GPa impliquant de recourir à de plus faibles épaisseurs. Pour chaque film, et en fonction du matériau utilisé, l'homme du métier saura déterminer une gamme d'épaisseurs permettant de réaliser la condition de flexion sans rupture mentionnée ci-dessus.

Lorsque la structure est soumise à une contrainte de flexion, la structure subit des contraintes en compression dans sa partie supérieure (réduction de la surface supérieure) et des contraintes en tension dans sa partie inférieure (augmentation de la surface inférieure), ou inversement suivant le sens de la flexion. Au milieu de la structure, les contraintes en compression et en tension se compensent, créant une surface où les contraintes mécaniques liées à la flexion sont nulles. Cette surface est nommée plan neutre, ou fibre neutre. Chaque élément d'interconnexion du composant électronique étant disposé à proximité de ce plan neutre, il subit peu de contraintes lors d'une flexion. Les interfaces de collage, de soudure et/ou de brasure sont ainsi préservées.

La proximité du plan neutre désigne ici une zone où les contraintes en compression et en tension sont très faibles, c'est-à-dire dans une zone correspondant à $\pm 20\%$, de préférence $\pm 10\%$, encore plus préférentiellement $\pm 5\%$, voire même $\pm 2\%$, de l'épaisseur totale de la structure par rapport à ce plan neutre. En d'autres termes, chaque élément d'interconnexion passe par un plan parallèle au plan neutre, situé à une distance de ce dernier inférieure ou égale à 20% de l'épaisseur totale de la structure, de préférence inférieure ou égale à 10% de cette épaisseur totale, encore plus préférentiellement inférieure ou égale à 5% voire même inférieure ou égale à 2% de cette épaisseur totale. Les distances sont mesurées, dans un sens ou dans l'autre, selon un axe orthogonal au plan de la structure électronique souple. Chaque piste électriquement conductrice, reliée à l'un des éléments d'interconnexion, est également au cœur de la structure. Elle n'est pas disposée en surface de la structure. Elle est proche du plan neutre, de sorte qu'elle n'est pas endommagée lors des sollicitations mécaniques, comme lors des contraintes en flexion.

La position de chaque élément d'interconnexion et de chaque piste électrique par rapport au plan neutre peut être contrôlée en ajustant l'épaisseur du premier film et/ou du deuxième film et/ou du troisième film.

Selon l'invention, la structure électronique souple comporte en outre au moins une couche de compensation, qui a pour fonction de déplacer localement la position du plan neutre selon l'axe de l'épaisseur de la structure. On peut ainsi positionner le plan neutre à un emplacement voulu, au plus près de chaque élément d'interconnexion. La couche de compensation est constituée de portions discrètes qui s'étendent chacune en vis-à-vis de l'une parmi l'au moins une piste électriquement conductrice, respectivement en vis-à-vis de l'un parmi l'au moins un composant électronique. Par en vis-à-vis, on entend que la portion de couche de compensation est face à la piste électriquement conductrice ou face au composant. De préférence, un projeté orthogonal de la portion de couche de compensation, dans le plan de la piste électriquement conductrice, respectivement du composant, ne dépasse pas latéralement relativement à ladite piste, respectivement relativement audit composant. La portion de couche de compensation peut être de même surface ou de surface différente, de même motif ou de motif différent, que la piste électriquement conductrice ou que le composant électronique. La surface désigne ici l'aire de la section dans un plan parallèle au plan des films. Chaque couche de compensation forme un film structuré, s'étendant dans un plan parallèle au plan de la structure électronique souple, et offrant une compensation non uniforme sur toute l'étendue de la structure électronique souple. En d'autres termes, la compensation est seulement locale selon l'étendue de la structure électronique souple.

La couche de compensation est réalisée, par exemple, dans un matériau dont le module de Young est proche de celui de la piste électrique, pour avoir des propriétés mécaniques (rigidité notamment) équivalentes. Plus généralement, les caractéristiques (matériau, surface, épaisseur, motif) de la couche de compensation sont choisies de manière à situer chaque élément de connexion, et si possible chaque piste électriquement conductrice, à proximité du plan neutre. Selon un mode de réalisation avantageux, le deuxième film comporte plusieurs composants électroniques de différentes surfaces, et l'une couche de compensation comprend une portion recouvrant le composant électronique de plus petite surface pour le rigidifier. De préférence, ladite couche de compensation a une rigidité supérieure à celle du composant électronique de plus petite surface, c'est-à-dire qu'elle a un module de Young supérieur à celui du composant électronique de plus petite surface. Une telle couche de compensation permet de maintenir le plan neutre au plus près du composant électronique enchâssé, en rigidifiant localement la structure. Ici également, la surface du composant électronique désigne l'aire d'une coupe de ce composant dans un plan parallèle au plan des premier, deuxième et éventuel troisième films.

L'un au moins des autres composants électroniques présente une plus grande surface, et n'est pas recouvert d'une portion de la couche de compensation. De préférence, la couche de compensation est constituée de la seule portion recouvrant le composant de plus petite surface.

En variante, une portion respective de la couche de compensation recouvre chaque composant électronique pour le rigidifier. Lesdites portions peuvent présenter des épaisseurs différentes.

Avantageusement, l'une parmi l'au moins une couche de compensation est disposée entre le deuxième film et le troisième film, constituée de portion(s) située(s) chacune en vis-à-vis de l'une parmi l'au moins une piste électriquement conductrice.

En complément ou en variante, l'une parmi l'au moins une couche de compensation recouvre le troisième film, et est constituée de portion(s) située(s) chacune en vis-à-vis de l'une parmi l'au moins une piste électriquement conductrice. Ladite couche de compensation s'étend alors du côté du troisième film opposé au composant électronique.

Selon une autre variante, l'une parmi l'au moins une couche de compensation est constituée de portion(s) située(s) chacune en vis-à-vis de l'une parmi l'au moins une piste électriquement conductrice, le premier film étant disposé entre la piste électriquement conductrice et la couche de compensation. En d'autres termes, la couche de compensation est sous le premier film, du côté opposé à l'au moins un composant électronique.

La structure selon l'invention peut comprendre deux couches de compensation distinctes l'une de l'autre. Par exemple, les deux couches de compensation sont constituées chacune de portions situées chacune en vis-à-vis d'une piste électriquement conductrice, de sorte que la même piste électriquement conductrice est alignée avec deux portions appartenant à deux couches de compensation distinctes. Les deux couches de compensation peuvent être nommées première couche de compensation, pour l'une, et couche de compensation additionnelle, pour l'autre. La couche de compensation additionnelle sera positionnée par l'homme du métier en fonction de la position de la première couche de compensation. La couche de compensation additionnelle peut être positionnée selon l'une des variantes décrites précédemment. Par exemple, la première couche de compensation peut être disposée entre le deuxième film et le troisième film, et la couche de compensation additionnelle peut être positionnée sous le premier film. En variante, deux couches de compensation peuvent être constituées, pour l'une, de portions situées chacune en vis-à-vis d'une piste électriquement conductrice, et pour l'autre de portions situées chacune en vis-à-vis d'un composant électronique.

Avantageusement, l'au moins une couche de compensation est en un même matériau que l'au moins une piste électriquement conductrice.

Avantageusement, le composant électronique a une épaisseur inférieure à 350 $\mu$m, et de préférence inférieure à 100 $\mu$m encore plus préférentiellement inférieure à 70 $\mu$m pour conserver les propriétés de souplesse de la structure. Il peut, par exemple, avoir une épaisseur de 20 $\mu$m à 50 $\mu$m ou encore de 70 $\mu$m à 100 $\mu$m.

Avantageusement, un via métallisé respectif, électriquement connecté à l'une parmi l'au moins une piste électriquement conductrice, s'étend dans le premier film de manière à connecter l'un parmi l'au moins un composant électronique à travers le premier film, le via métallisé étant rempli par le deuxième polymère. La piste électriquement conductrice peut ainsi être interconnectée électriquement avec l'extérieur grâce au via métallisé.

La structure selon l'invention peut comporter en outre au moins une pointe métallisée, chacune électriquement connectée à l'une parmi l'au moins une piste électriquement conductrice par l'intermédiaire du via correspondant, et faisant saillie à partir du premier film

du côté opposé à ladite piste électriquement conductrice, la pointe étant remplie par le deuxième polymère.

La structure peut comprendre au moins une tranchée de scellement, chacune entourant l'un parmi l'au moins un composant électronique, le polymère du troisième film remplissant la tranchée de scellement.

Avantageusement, le premier polymère, le troisième polymère et, de préférence, le deuxième polymère, sont identiques.

Avantageusement, au moins un trou traversant s'étend dans le premier film et/ou dans le troisième film, chacun de manière à rendre accessible l'un parmi l'au moins un composant électronique. Les trous traversants forment des évents qui permettent aux composants d'être exposés à l'atmosphère avoisinante. Cela peut présenter un intérêt par exemple pour des applications où le dispositif souple doit interagir avec son environnement extérieur, comme par exemple pour mesurer la perte hydrique d'un individu.

Avantageusement, le composant électronique est un circuit intégré à application spécifique, un capteur, un actuateur, un stimulateur, une microbatterie, ou une puce RFID. De nombreux composants de différentes natures peuvent être intégrés.

L'objectif poursuivi est également atteint par un procédé de préparation d'une structure électronique souple telle que définie précédemment, ledit procédé comprenant les étapes successives suivantes :

a) fourniture d'un substrat,
b) formation du premier film en un premier polymère ou en verre, sur le substrat,
c) formation de l'au moins une piste électriquement conductrice sur le premier film,
d) formation du deuxième film en un deuxième polymère dans lequel est disposé au moins un composant électronique, chaque composant électronique étant électriquement connecté avec l'une parmi l'au moins une piste électriquement conductrice, par un élément d'interconnexion respectif,
e) éventuellement, formation du troisième film en un troisième polymère ou en verre,
f) séparation du substrat de la structure électronique souple.

Le procédé de l'invention comprend également au moins une étape de formation d'au moins une couche de compensation.

Le procédé de l'invention est simple à mettre en œuvre. Il fait appel à des procédés classiques utilisés en microélectronique et dans le domaine de l'assemblage et l'encapsulation de composants électroniques. Les différents films en polymère ou en verre peuvent être formés dans une gamme de température allant de la température ambiante (20-25°C) jusqu'à, par exemple, 350°C, en fonction de la nature des polymères. Le procédé est facilement transposable à une échelle industrielle.

Avantageusement, l'étape d) est réalisée en :

- formant un film plein constitué du matériau du deuxième film,
- gravant le film plein, de manière à former au moins une cavité, chaque cavité rendant accessible l'une parmi l'au moins une piste électriquement conductrice,
- reportant un composant électronique respectif dans chaque cavité et en connectant chaque composant électronique avec l'une au moins parmi l'au moins une piste électriquement conductrice, chaque connexion étant réalisée par l'intermédiaire d'un élément d'interconnexion respectif,
- éventuellement, amincissant l'un au moins composant électronique.

Selon une variante, l'étape d) est réalisée en :

- reportant et connectant au moins un composant électronique, chacun sur l'une au moins parmi l'au moins une piste électriquement conductrice, chaque connexion étant réalisée par l'intermédiaire d'un élément d'interconnexion respectif,
- éventuellement, amincissant l'un au moins composant électronique.
- formant le deuxième film sur l'au moins un composant électronique et sur le premier film.

L'amincissement est mis en œuvre par gravure ou meulage. Dans les deux variantes, l'amincissement éventuel est réalisé en cours de préparation de la structure souple, sur toute la structure. L'amincissement d'un composant électronique préalablement positionné à son emplacement final dans la structure simplifie le procédé selon l'invention, en comparaison avec un amincissement préalable au report sur la structure finale. Lorsque la structure souple comporte plusieurs composants électroniques, l'amincissement des composants est réalisé de façon collective. L'amincissement collectif est plus facile à réaliser qu'une pluralité d'amincissements distincts sur des éléments uniques, comme dans l'art antérieur. Dans la première variante, le deuxième film peut servir de couche d'arrêt d'une gravure réalisant l'amincissement.

Avantageusement, entre l'étape a) et l'étape b), une couche sacrificielle est déposée sur le support et lors de l'étape f), la couche sacrificielle est gravée pour séparer le support de la structure électronique souple.

Avantageusement, avant l'étape b), l'une parmi l'au moins une couche de compensation est formée sur le substrat, et/ou, après l'étape d), l'une parmi l'au moins une couche de compensation est formée sur le deuxième film et/ou, après l'étape e), l'une parmi l'au moins une couche de compensation est formée sur le troisième film.

Avantageusement, le premier polymère, respectivement le troisième polymère, est un polyimide, un polysiloxane, un parylène, un polymère thermoplastique.

Le substrat comporte avantageusement des renfoncements en forme de pointe, formant des empreintes pour former des pointes métallisées faisant saillie à partir du premier film, du côté opposé à la piste électriquement conductrice.

## BRÈVE DESCRIPTION DES DESSINS

La présente invention sera mieux comprise sur la base de la description qui va suivre et des dessins en annexe sur lesquels :

- la figure 1A est une représentation schématique, en coupe et vue de profil, d'une structure électronique souple enroulée autour d'une surface courbe,
- la figure 1B est une représentation schématique, en coupe et vue de profil, d'une structure électronique souple selon un premier mode de réalisation de l'invention,
- la figure 2 est une représentation schématique, en coupe et vue de profil, d'une structure électronique souple selon un deuxième mode de réalisation de l'invention,
- la figure 3 est une représentation schématique, en coupe et vue de profil, d'une structure électronique souple selon un troisième mode de réalisation de l'invention,
- la figure 4 est une représentation schématique, en coupe et vue de profil, d'une structure électronique souple selon un quatrième mode de réalisation de l'invention,
- les figures 5A et 5B sont des représentations schématiques, en coupe et vue de profil, de deux variantes d'une structure électronique souple selon un cinquième mode de réalisation de l'invention,
- la figure 6 est une représentation schématique, en coupe et vue de profil, d'une structure électronique souple selon un sixième mode de réalisation de l'invention,
- la figure 7 est une représentation schématique, en coupe et vue de profil, d'une structure électronique souple selon un septième mode de réalisation de l'invention,
- la figure 8 est une représentation schématique, en coupe et vue de profil, d'une structure électronique souple selon un huitième mode de réalisation de l'invention,
- la figure 9 est une représentation schématique, en coupe et vue de profil, d'une structure électronique souple selon un neuvième mode de réalisation de l'invention,
- la figure 10 est une représentation schématique d'un composant électronique, vue de dessus, connecté à des pistes électriquement conductrices selon un mode de réalisation particulier de l'invention,
- les figures 11A à 11M représentent de manière schématique différentes étapes du procédé de réalisation d'une structure électronique souple, selon un mode de réalisation particulier de l'invention,

- les figures 12A à 12B représentent de manière schématique différentes étapes du procédé de réalisation d'une structure électronique souple, selon un autre mode de réalisation particulier de l'invention,
- les figures 13A à 13B représentent de manière schématique différentes étapes du procédé de réalisation d'une structure électronique souple, selon un autre mode de réalisation particulier de l'invention.

Les différentes parties représentées sur les figures ne le sont pas nécessairement selon une échelle uniforme, pour rendre les figures plus lisibles.

Les différentes possibilités (variantes et modes de réalisation) doivent être comprises comme n'étant pas exclusives les unes des autres et pouvant se combiner entre elles.

## EXPOSÉ DÉTAILLÉ DE MODES DE RÉALISATION PARTICULIERS

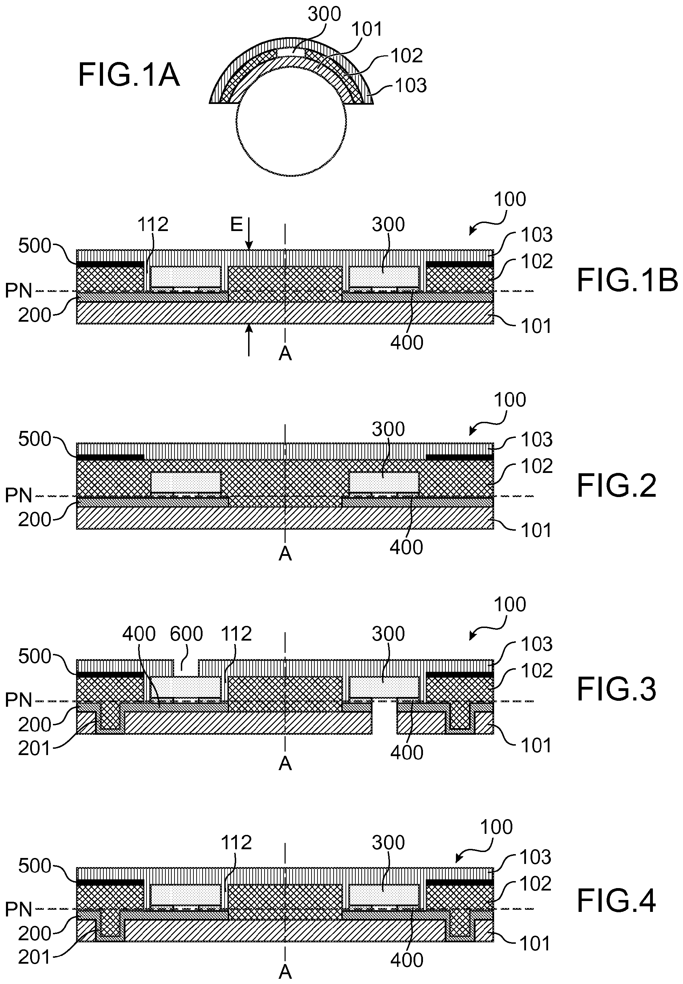

On se réfère tout d'abord aux figures 1 à 10 qui représentent une structure électronique souple 100 selon différents modes de réalisation.

L'homme du métier pourra combiner les différents modes de réalisation les uns avec les autres de différentes manières.

### Structure électronique souple :

Comme représenté sur la figure 1A, la structure 100 est flexible, elle peut, par exemple, s'enrouler autour d'une surface courbe sans qu'il n'y ait de rupture ou de cassures dans la structure. La structure est conformable. Elle peut être fléchie jusqu'à avoir un rayon de courbure inférieur ou égal à 1000 mm, de préférence inférieur ou égal à 200 mm, ce rayon de courbure pouvant atteindre 10 mm, et même 5 mm, voire jusqu'à 2 mm sans que la structure 100 ne soit endommagée.

La structure 100 comprend en particulier un premier film 101, un deuxième film 102, un troisième film 103 optionnel, au moins un composant électronique 300 électroniquement connecté à au moins une piste électriquement conductrice 200 respective, et au moins une couche de compensation 500.

Pour plus de lisibilité, seuls le premier film 101, le deuxième film 102 et le troisième film 103 film ainsi qu'un composant électronique 300 sont représentés en figure 1A.

La structure électronique souple 100 peut être directement portée par une personne, par exemple sur un poignet, un bras ou un torse. La structure électronique 100 peut être utilisée pour des applications médicales ou de bien-être par exemple. A titre illustratif, la structure 100 peut faire partie intégrante d'un dispositif de mesure de température, de rythme cardiaque, d'actimétrie, ou de dégazage de la peau (sueur), ou encore d'un dispositif de stimulation électrique ou optique, ou d'un dispositif d'administration de médicament. La structure peut être enroulée autour d'un cathéter, par exemple. La structure électronique souple 100 peut être directement reportée sur tout type d'objet aux formes plus ou moins arrondies.

La structure 100 a une épaisseur comprise entre 20 $\mu$m et 400 $\mu$m, de préférence entre 50 $\mu$m et 150 $\mu$m et encore plus préférentiellement de l'ordre de 100 $\mu$m.

Dans tout le texte, le terme « épaisseur » désigne une dimension selon un axe perpendiculaire à l'empilement des films en polymère ou en verre.

On définit également le plan de la structure électronique souple comme étant un plan orthogonal à l'axe de l'épaisseur, parallèlement auquel s'étendent chacun des films en polymère ou en verre.

Le plan neutre de la structure (aussi appelé fibre neutre) est représenté par le trait en pointillé PN sur les différentes figures. Les contraintes mécaniques lors d'une flexion sont minimales dans ce plan et les éléments disposés dans ce plan conservent leur intégrité. Selon l'invention, le plan neutre se trouve au niveau des connexions électriques entre les composants électroniques 300 et les pistes électriquement conductrices 200.

### Le premier film 101 :

Le premier film 101, aussi appelé film inférieur, est flexible. On entend par flexible que le film peut subir une flexion d'un rayon de courbure inférieur ou égal à 1000 mm et de préférence inférieur ou égal à 200 mm sans rupture.

Le premier film 101 est en polymère ou en verre. Par polymère, on entend de préférence, ici et dans la suite, un homopolymère ou un copolymère. A titre illustratif, le polymère peut être choisi parmi la liste non exhaustive suivante (on a indiqué le module de Young entre parenthèses): un polyimide (de 2 à 8 GPa, par exemple 3,2 Pa), un siloxane polymérisé (de 0,05 à 0,5 GPa, par exemple 0,15 GPa) tel que du silicone (moins de 0,1 GPa) ou un polymère siloxane SINR$^{(\text{TM})}$, du parylène (2,4 à 3 GPa), un thermoplastique tel que le polyéthylène (200 à 700MPa), du poly-téréphtalate d'éthylène ou PET (2800-3100MPa), du poly(naphtalate d'éthylène) ou PEN (500-1500MPa), etc. Son épaisseur va de 5 $\mu$m à 150 $\mu$m, de préférence de 10 $\mu$m à 75 $\mu$m, et encore plus préférentiellement de 10 $\mu$m à 30 $\mu$m. On remarque que lorsque le premier film est en verre, il présente un module de Young plus élevé (de 69 à 72 GPa) de sorte qu'il présente alors une épaisseur située dans la partie basse des gammes d'épaisseur indiquées ci-dessus.

### Le deuxième film 102 :

Le deuxième film 102 (aussi appelé film interne) est constitué d'un matériau flexible en polymère. Il est, avantageusement, constitué d'un matériau apte à remplir des vias ou des tranchés. Cela peut être un thermoplastique ou un polymère siloxane SINR$^{(\text{TM})}$. En variante,

il peut s'agir également d'un polyimide, un siloxane polymérisé tel du silicone ou du parylène. L'épaisseur de ce film va de 5 $\mu$m à 150 $\mu$m, de préférence de 10 $\mu$m à 75 $\mu$m, et encore plus préférentiellement de 10 $\mu$m à 30 $\mu$m.

### Le troisième film 103 (optionnel) :

Le troisième film 103 (aussi appelé film supérieur) est élaboré en un matériau flexible. Le matériau du troisième film est un polymère ou un film de verre. Cela peut être un polyimide, un siloxane polymérisé tel que du silicone ou un polymère siloxane SINR$^{(\text{TM})}$, un thermoplastique, du parylène ou un verre. Il sera avantageusement dans un matériau apte à remplir des vias ou des tranchées. L'épaisseur de ce film va de 5 $\mu$m à 150 $\mu$m, de préférence de 10 $\mu$m à 75 $\mu$m, et encore plus préférentiellement de 10 $\mu$m à 30 $\mu$m.

Comme représenté sur les figures 1A et 1B, le deuxième film 102 peut avoir une épaisseur identique à celle du composant 300 à encapsuler. Par identique, on entend que la différence d'épaisseur est inférieure ou égale à 30% de l'épaisseur du composant 300 (épaisseur dans la structure souple selon l'invention, après éventuel amincissement).

Selon des variantes, le deuxième film 102 peut avoir une épaisseur inférieure ou supérieure (figure 2) à l'épaisseur du composant électronique.

Lorsque le deuxième film 102 a une épaisseur supérieure à celle du composant électronique 300 et qu'il le recouvre, la structure peut ne pas comprendre de troisième film 103. Par exemple, dans ce mode de réalisation, le deuxième film 102 peut avoir une épaisseur environ deux fois plus grande que celle du premier film 101, de manière à maintenir le plan neutre au niveau de la connexion entre le composant électronique 300 et la piste électriquement conductrice 200, sans qu'il soit nécessaire d'ajouter un troisième film au-dessus du deuxième film.

Les trois films (premier 101, deuxième 102 et troisième 103 films) peuvent être dans des matériaux différents ou identiques.

De préférence, le premier film 101 et le troisième film 103 sont en un même matériau. Les propriétés mécaniques de la structure 100 (souplesse notamment) sont ainsi mieux équilibrées. Le matériau du premier film 101 et/ou du troisième film 103 peuvent être des matériaux biocompatibles, par exemple en polysiloxane.

Dans un mode particulier de réalisation, représenté sur la figure 3, des trous traversants 600 sont disposés dans le premier film 101, et/ou dans le troisième film 103 ou le deuxième film, de manière à rendre accessible chacun l'un des composants électroniques 300, depuis l'extérieur. Les trous traversants 600 forment des évents qui permettent aux composants 300 d'être exposés à l'atmosphère avoisinante. Cela peut présenter un intérêt par exemple pour des applications où le dispositif souple doit interagir avec son environnement extérieur, comme par exemple pour mesurer la perte hydrique d'un individu.

**Les pistes électriques 200** :

Les pistes électriquement conductrices 200, ou pistes électriques 200, sont disposées au sein de la structure 100, entre le premier film 101 et le deuxième film 102, au plus près du plan neutre.

Les pistes électriques 200 sont électriquement conductrices. Elles peuvent être métalliques, par exemple, en Cu, Ag, Au, Al, W, Ni, Pt, Ti ou Ru. Dans le cas d'une structure devant être biocompatible, par exemple pour des applications médicales, on choisira un métal noble, comme par l'exemple, l'or ou le platine. Les pistes électriques 200 peuvent également être réalisées à partir d'une encre chargée en particules métalliques comme celles utilisées dans l'électronique organique.

L'épaisseur des pistes 200 pourra être de 50 nm à 5 $\mu$m, et de préférence de 100 nm à 2 $\mu$m.

Comme représenté sur les figures 3 à 9, des vias 201 peuvent être réalisés dans le premier film 101. Les vias traversent le premier film 101 et sont métallisés (c'est-à-dire recouverts d'une couche de métal, électriquement conductrice). Le matériau du deuxième film 102 se prolonge dans les vias 201 et remplit les vias 201, ce qui améliore le maintien mécanique de la structure 100.

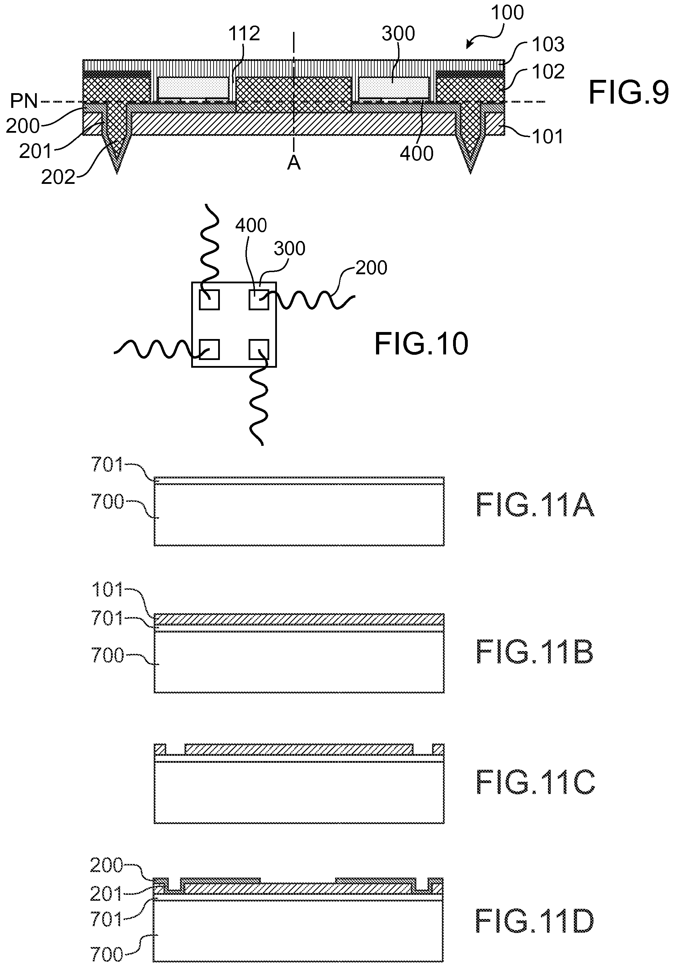

Comme représenté sur la figure 9, la structure souple 100 peut également comprendre une ou plusieurs pointes 202, ou saillies, métallisées, faisant saillie à partir du premier film 101 dans la direction opposée aux pistes électriquement conductrices. Les pointes 202 sont connectées aux pistes électriques 200 par les vias 201. En particulier, chaque pointe 202 s'étend dans la continuité d'un via 201, du côté opposé aux pistes électriques, sous le premier film 101. Les pointes 201 sont remplies chacune par le matériau du deuxième film 102.

Les épaisseurs des films mentionnées ci-avant ne prennent pas en considération les éventuels vias ni les éventuelles pointes.

Dans une variante représentée sur la figure 10, les pistes électriques 200 sont sous forme de serpentin de façon à leur conférer une certaine élasticité et donc de contribuer mécaniquement au comportement souple de la structure 100. Lors des contraintes en flexion, les pistes électriques pourront s'étirer. L'agencement des différentes pistes d'une même structure souple 100 présente avantageusement une symétrie telle que celle illustrée en figure 10, pour participer à une symétrie de la structure 100. Ici, il s'agit d'une symétrie axiale centrée sur le centre géométrique de la structure 100.

**Le composant électronique intégré 300** :

Le composant intégré 300 dans la structure 100 peut être choisi parmi un ASIC (c'est-à-dire circuit intégré à application spécifique), un capteur, un actuateur, un stimulateur, une batterie, une puce RFID (c'est-à-dire identification radiofréquence), ou encore un composant passif. La face du composant en contact avec les plots de connexion électrique 400 est en face du premier film 101, pour pouvoir connecter électriquement chaque plot de connexion à l'une piste électriquement conductrice 200. Le composant 300 est, par exemple, en silicium.

Une fois intégré dans la structure, le composant électronique 300 a une épaisseur comprise entre quelques dizaines, voire quelques centaines de micromètres, et une dizaine de micromètres. Il a par exemple une épaisseur comprise entre 10 $\mu$m et 350 $\mu$m. Son épaisseur est par exemple comprise entre 100 $\mu$m et 150 $\mu$m. En variante, il a une épaisseur inférieure à 100 $\mu$m, et de préférence inférieure à 50 $\mu$m, par exemple de 20 $\mu$m à 50 $\mu$m, pour maximiser les propriétés de souplesse de la structure.

Comme représenté sur les figures 1B et 3 à 9, le composant électronique 300 est, de préférence, entouré par une tranchée de scellement 112. La tranchée de scellement 112 sépare le deuxième film 102 du composant 300. La largeur de la tranchée 112 peut aller de 10 $\mu$m à 500 $\mu$m, et de préférence de 30 $\mu$m à 100 $\mu$m. La tranchée de scellement 112 est remplie par le matériau du troisième film 103 s'il existe, par le matériau du deuxième film 102 sinon.

La structure 100 peut comprendre un ou plusieurs composants 300, de même surface ou de surfaces différences, de même nature ou de natures différentes. Par exemple, on peut avoir une puce RFID et un capteur.

De préférence, mais de manière optionnelle, et comme représenté sur les figures 1A à 9, la structure 100 comprend plusieurs composants 300 et présente un plan de symétrie (plan A), ou un axe de symétrie (axe A). On respectera au mieux cette symétrie pour contrôler la position du plan neutre. Le plan A, respectivement l'axe A passe par le centre de la structure 100, entre au moins deux composants électroniques, et s'étend orthogonal aux plans des films 101, 102 et 103.

**L'élément d'interconnexion 400 entre la piste électriquement conductrice 200 et le composant électronique intégré 300** :

L'élément d'interconnexion 400, ou plot d'interconnexion, connecte électriquement et mécaniquement chaque piste électriquement conductrice 200 avec un composant 300. L'élément d'interconnexion 400, la piste électrique 200 et le composant électronique 300 sont solidaires.

Un même composant électronique 300 peut être relié à une ou plusieurs pistes électriques 200. Par exemple sur la figure 10 le composant 300 est relié à quatre pistes électriquement conductrices 200 via quatre éléments d'interconnexion 400.

L'élément d'interconnexion 400 est électriquement conducteur. Par exemple, il peut être réalisé à partir d'une brasure fusible à base d'étain ou de plomb, par exemple, telle que SnAg, SnPb ou SnAgCu. Il peut également s'agir d'un bossage, de préférence en or, plus connu sous le nom de « stud bump » ou « accu bump ». Il peut également s'agir d'une colle conductrice. Il peut également s'agir d'une encre conductrice, par exemple à base d'argent. Il peut aussi être fait pour partie d'un élément conducteur, tel un pilier métallique, appelé micro-insert, micro-tube (ou « pilar »), selon les techniques utilisées pour connecter le composant électronique 300 à la piste électrique 200.

L'élément d'interconnexion 400 a une épaisseur allant de 0,5 $\mu$m à 70 $\mu$m. De préférence, l'élément d'interconnexion ne présente une épaisseur supérieure à 10 $\mu$m que s'il s'agit d'un « stud bump ».

Selon l'invention, l'élément d'interconnexion 400 passe par un plan dont la distance au plan neutre est inférieure ou égale à 20% d'une épaisseur totale E de la structure 100. Ladite distance est mesurée dans un sens ou dans l'autre, selon un axe orthogonal au plan selon lequel s'étend la structure souple.

Lorsque l'élément d'interconnexion 400 est un bossage de type « studbump », il présente une épaisseur élevée, supérieure à 10 $\mu$m. Un tel élément d'interconnexion 400 est fixé initialement à l'un parmi la piste électrique 200 et le composant 300, par fusion, puis fixé à l'autre parmi la piste électrique 200 et le composant 300, par simple thermo-compression. La fixation réalisée par thermo-compression est la plus fragile des deux fixations. Par conséquent, on positionne au plus proche du plan neutre la face de l'élément d'interconnexion qui est fixée par thermo-compression. La fixation par thermo-compression peut se trouver à l'interface entre l'élément d'interconnexion 400 et la piste électrique 200, ou à l'interface entre l'élément d'interconnexion 400 et le composant électronique 300.

A titre illustratif seulement, dans le cas d'un empilement comprenant une couche supérieure et une couche inférieure, empilées l'une sur l'autre, et homogènes, la position du plan neutre peut être calculée avec la formule décrite dans les articles respectifs de Suo et al. ("Interface crack between two elastic layers", International Journal of Fracture 43:1-18, 1990) et de Eberl et al. ("Mechanical Characterization of Coatings Using Microbeam Bending and Digital Image Correlation Techniques", Experimental Mechanics DOI 10.1007/s11340-008-9187-4) :

$$\frac{h_0}{h_{inf}} = \frac{1 + \dfrac{2.E_{sup}.h_{sup}}{E_{inf}.h_{inf}} + \dfrac{E_{sup}.h_{sup}^2}{E_{inf}.h_{inf}^2}}{2.\left(1 + \dfrac{E_{sup}.h_{sup}}{E_{inf}.h_{inf}}\right)}$$

avec :

$h_0$ la position du plan neutre,
$h_{sup}$ et $E_{sup}$, respectivement, l'épaisseur et le module de Young de la couche supérieure,
$h_{inf}$ et $E_{inf}$, respectivement, l'épaisseur et le module de Young de la couche inférieure.

Par exemple, dans le cas d'un empilement ayant :

- une couche supérieure en silicium avec les caractéristiques suivantes : $h_{sup}$=45 $\mu$m, $E_{sup}$=130 GPa ,
- une couche inférieure en polymère avec les caractéristiques suivantes : $h_{inf}$=100 $\mu$m, $E_{inf}$=2,5 GPa,

la position du plan neutre $h_0$ est à 120 $\mu$m, c'est-à-dire que le plan neutre est dans la couche supérieure.

Le déplacement du plan neutre peut être réalisé, par exemple, par l'ajout d'une couche métallique en nickel (10 $\mu$m d'épaisseur et 214 GPa de module de Young) ou en ruthénium (5 $\mu$m d'épaisseur et 447 GPa de module de Young), sous l'empilement, i.e. en contact avec la couche inférieure. La nouvelle position du plan neutre est, respectivement, à 110 $\mu$m et à 105 $\mu$m.

## La couche de compensation 500 :

Comme représenté sur les figures, la structure comprend en outre une couche de compensation 500, aussi appelée couche compensatrice, pour rigidifier localement la structure dans sa région la plus souple, et ainsi modifier la position du plan neutre de la structure.

La couche de compensation 500 est une couche constituée de portions discrète espacées les unes des autres.

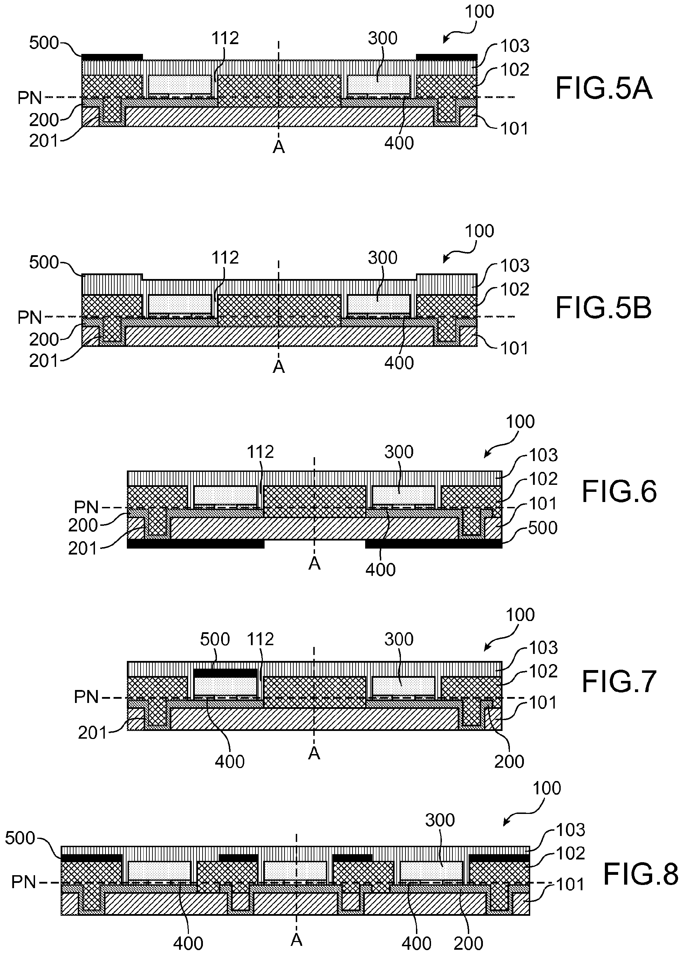

La couche de compensation 500 permet de rigidifier, par exemple, la partie supérieure de la structure tout comme l'au moins une piste électrique 200 rigidifie la partie inférieure de la structure (figures 1A à 5B). Elle peut également permettre de rigidifier encore plus la partie inférieure de la structure (figure 6). La couche de compensation 500 et l'au moins une piste électrique 200 ont de préférence un même module de Young. En variante, on peut utiliser pour la couche de compensation 500 un matériau à plus fort module de Young que celui utilisé dans la piste 200, de façon à réduire l'épaisseur de la couche de compensation 500.

L'homme du métier choisira les matériaux et les épaisseurs de l'au moins une piste électrique 200 et de la couche de compensation 500 de manière à situer l'au moins un élément d'interconnexion 400 au plus proche du plan neutre. La couche de compensation 500 et l'au moins une piste électrique 200 peuvent être en un même matériau et/ou avoir la même épaisseur.

La couche de compensation 500 est, par exemple, métallique. Elle peut être en Cu, Ag, Au, Al, W, Ni, Pt ou Ti. Pour des applications nécessitant une biocompatibilité, on choisira un métal noble comme par l'exemple, l'or ou le platine.

Il peut également s'agir d'une encre chargée, comme celles utilisées dans l'électronique organique, ou d'un polymère.

L'épaisseur de la couche de compensation pourra être comprise entre 100 nm et 10 $\mu$m, par exemple entre 0,5 $\mu$m et 10 $\mu$m ou entre 100 nm et 5 $\mu$m, et de préférence entre 0,5 $\mu$m et 2 $\mu$m.

Selon un premier mode de réalisation, la couche de compensation 500 est constituée de portion(s) disposée(s) chacune au-dessus de l'une des pistes électriquement conductrices 200, chacune en vis-à-vis de l'une piste électrique 200. La couche de compensation 500 s'étend alors du côté du deuxième film 102 opposé au premier film 101.

Selon une première variante représentée sur les figures 1B à 4, la structure 100 comprend le troisième film 103, et la couche de compensation 500 et l'au moins une piste électriquement conductrice 200 sont disposées de part et d'autre du deuxième film 102. La couche de compensation 500 s'étend alors directement sur le deuxième film 102, du côté opposé au premier film 101. Par « directement », on entend en contact physique direct, sans couche intercalaire.

Selon une autre variante, comme représentée sur les figures 5A et 5B, la structure 100 comprend le troisième film 103, et la couche de compensation 500 et l'au moins une piste électriquement conductrice 200 sont disposées de part et d'autre de l'ensemble formé par le deuxième film 102 et le troisième film 103. Autrement dit, la couche de compensation 500 est disposée directement sur le troisième film 103, du côté opposé au composant 300. De préférence, et comme représenté en figures 5A et 5B, la couche de compensation 500 est constituée de portion(s) ne recouvrant pas le composant électronique 300. En variante, la première couche de compensation 500 peut être constituée de portion(s) ayant chacune la même taille que la piste électriquement conductrice 200 située en vis-à-vis. On pourra, avantageusement, choisir des éléments de même forme.

Dans la variante illustrée en figure 5A, la couche de compensation 500 est constituée d'un matériau distinct de celui du troisième film.

Dans la variante illustrée en figure 5B, la couche de compensation 500 est constituée de surépaisseurs locales dans une couche en verre qui forme à la fois le troisième film 103 et la couche de compensation 500. On a par exemple une épaisseur de l'ordre de 10 $\mu$m à l'aplomb des composants électroniques 300, et une épaisseur d'environ 100 $\mu$m à l'aplomb des pistes 200. En tout état de cause, l'épaisseur de verre à l'aplomb des pistes est au moins 5 fois supérieure à l'épaisseur de verre à l'aplomb des pistes 200. Cette variante est réalisée par gravure d'une couche de verre déposée sur le deuxième film 102 (gravure chimique et lithographie, par exemple).

Dans les deux variantes, le troisième film est délimité par une surface plane, du côté opposé au deuxième film.

Selon une autre variante, non représentée, la structure 100 ne comporte pas le troisième film 103, et la couche de compensation 500 s'étend directement sur le deuxième film 102, formée de portion(s) située(s) chacune en vis-à-vis de l'une piste électrique 200. De préférence, chaque portion de la couche de compensation 500 ne recouvre pas l'au moins un composant 300.

Selon un deuxième mode de réalisation comme représenté sur la figure 6, la couche de compensation 500 est disposée en-dessous du premier film 101, constituée de portion(s) située(s) chacune en vis-à-vis de l'une piste électrique 200. La couche de compensation 500 s'étend alors du côté du premier film 101 opposé au deuxième film 102. De préférence, la couche de compensation 500 est constituée de portion(s) qui s'étendent chacune jusqu'en dessous de l'un composant électronique 300. Selon une variante non représentée, la couche de compensation est disposée en-dessous du premier film 101, constituée de portion(s) située(s) chacune en vis-à-vis de l'un composant électronique 300.

Dans chacun de ces modes de réalisation, la couche de compensation 500, lorsqu'elle dépasse relativement à l'empilement de films 101, 102, 103, n'est pas prise en compte pour déterminer l'épaisseur totale de la structure 100.

Selon une variante non représentée, la structure 100 peut comprendre au moins une couche de compensation additionnelle qui peut être en un même matériau que l'au moins une piste électriquement conductrice 200.

Lorsque la structure 100 comprend au moins deux composants 300 de surfaces différentes, la position du plan neutre est modifiée, par rapport à la position qu'il aurait avec deux composants 300 de même taille. Par surface, on entend la dimension orthogonale à l'axe d'empilement des films 101, 102 et 103. Pour compenser cet effet, on peut rigidifier le composant 300 de plus petite surface par ajout d'une couche de compensation 500 constituée d'une portion recouvrant uniquement ce composant 300 de plus petite surface (figure 7). En variante, la couche de compensation 500 est constituée de plusieurs portions recouvrant chacune l'un parmi plusieurs composants de plus petite surface. L'un au moins des composants n'est alors recouvert d'aucune portion de la couche de compensation 500. Dans l'exemple illustré en figure 7, le composant de plus petite surface est directement recouvert d'une portion de la couche de compensation, du côté opposé au premier film 101. Selon une variante non représentée, une portion discrète de la couche de compensation s'étend en vis-à-vis du composant de plus petite surface, sous le premier film, sur le troisième film s'il existe, ou sur le deuxième film en l'absence de troisième film. Ce mode de réalisation peut être combiné, ou non, à chacun des modes de réalisation et variantes d'agencement d'une couche de compensation tels que décrits ci-dessus.

La figure 8 illustre une autre solution pour compenser cet effet, qui consiste à restituer à la structure 100 un plan de symétrie A passant par le composant de plus grande surface. Dans le cas où la structure 100 comporte seulement deux composants 300, on ajoute alors un troisième composant identique au composant de plus petite surface, le troisième composant étant disposé de manière symétrique au composant de plus petite surface par rapport au plan A. On corrigera aussi, dans ce cas, le nombre et la position des vias 201 de façon à rendre la structure 100 la plus symétrique possible. En particulier, le composant central peut être relié électriquement à deux fois plus de vias que les autres composants, pour de simples raisons de symétrie. De la même manière, le composant central est positionné ici de manière centrale au-dessus d'une piste électrique correspondante, laquelle est rigidifiée par une portion de couche de compensation ouverte au centre.

## Procédé de réalisation :

Le procédé de réalisation d'une telle structure 100 va maintenant être décrit. Le procédé comporte les étapes successives suivantes :

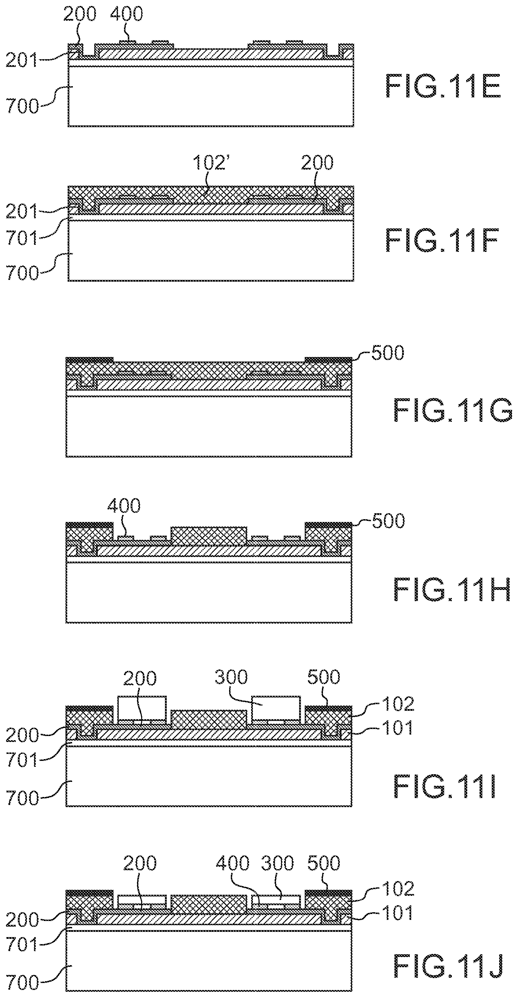

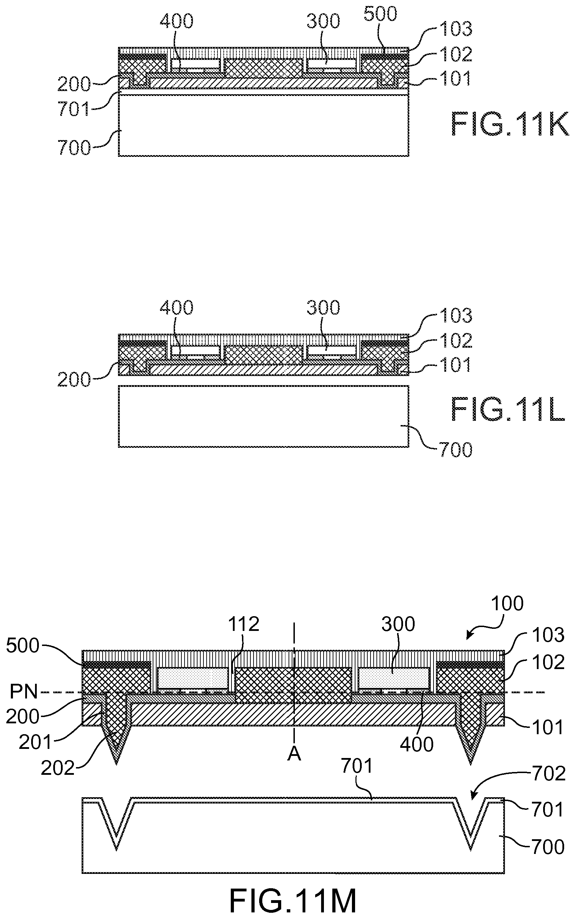

   a) fourniture d'un substrat 700 (figure 11A),
   b) formation du premier film 101 en un premier polymère ou en verre (figure 11B),
   c) formation de l'au moins une piste électriquement conductrice 200 sur le premier film 101 (figure 11D),
   d) formation du deuxième film 102 dans lequel est encapsulé l'au moins un composant électronique 300, électriquement connecté avec l'une piste électriquement conductrice 200, par l'élément d'interconnexion 400 respectif (figures 11F à 11J),
   e) éventuellement, formation du troisième film 103 (figure 11K),
   f) séparation du substrat 700 de la structure électronique souple 100 (figure 11L).

Le substrat 700 est un support rigide et temporaire. Il sert de base pour élaborer la structure 100 et sera retiré une fois la structure achevée. Il ne fait pas partie de la structure souple finale 100. Le support 700 est, avantageusement, plan. Le support 700 est, par exemple, en silicium ou en verre. Le support n'a pas besoin d'être monocristallin et peut donc être constitué de silicium polycristallin, dont le coût est bien moindre.

Selon une variante illustrée en figure 11M, le substrat peut contenir une ou plusieurs cavités 702, ou renfoncements, réalisant ainsi une empreinte pour l'au moins une pointe métallisée 202 faisant saillie à partir du premier du film 101. Les renfoncements 702, avantageusement pyramidaux peuvent être réalisés par exemple par gravure chimique dans du silicium monocristallin.

Le substrat 700 comprend, de préférence, une couche sacrificielle 701 (aussi appelée couche d'arrêt) qui pourra être gravée pour libérer la structure 100 du substrat 700, ou servir de couche d'arrêt, dans le cas d'un amincissement mécanique du substrat 700 (étape F). La couche sacrificielle 701 peut être en un matériau facile à graver chimiquement. Lorsque les films 101 et 103 sont en polymère, il s'agit de préférence d'un métal comme du titane qui se grave très rapidement avec de l'acide fluorhydrique.

La couche sacrificielle 701 peut également être une résine ou un film adhésif, ou un polymère photosensible, ou encore un empilement de couches dont l'adhésion peut être rompue mécaniquement ou thermiquement.

La couche sacrificielle 701 peut être déposée à la tournette, par dépôt physique en phase vapeur ou par laminage.

Selon une variante non représentée, avant l'étape b), une couche de compensation 500 peut être formée sur le substrat 700, ou sur la couche sacrificielle si le substrat 700 en comprend une, de manière à être directement en contact avec le premier film 101. La couche de compensation 500 peut être constituée de portions discrètes s'étendant chacune en vis-à-vis de l'un composant électronique, sous la piste électriquement conductrice associée, et/ou en vis-à-vis de l'une piste électriquement conductrice. La couche de compensation 500 peut être déposée et gravée par des procédés de microélectronique connus de l'homme du métier.

Lors de l'étape b), le premier film 101 est formé. Le premier film 101 peut être déposé à l'état liquide par dépôt à la tournette (« spin-coating ») sur le substrat 700 puis durci thermiquement, par exemple par évaporation. Il peut également être déposé par laminage d'un film sec. Un ou plusieurs vias 201 peuvent être réalisés dans le premier film 101 (figure 11C). Les vias 201 peuvent être gravés par des procédés de microélectronique connus de l'homme du métier: gravure plasma, gravure laser, gravure ionique, gravure chimique. La largeur des vias 201 peut aller de 10 à 500 μm, de préférence 80 μm à 120 μm.

La piste électriquement conductrice 200 et l'élément d'interconnexion 400 sont ensuite réalisés (étape c). Ce dernier permet d'interconnecter les composants par brasure collage ou compression mécanique lors de l'étape d). Lorsque le premier film 101 comporte des vias 201, respectivement des vias et des pointes en saillie, ils seront métallisés lors de cette étape. Les pistes électriques 200 sont déposées et gravées par des procédés de microélectronique connus de l'homme du métier.

Lors de l'étape d), le deuxième film 102, dans lequel est encapsulé le composant électronique 300, est formé. Le deuxième film 102 est, de préférence, déposé à l'état liquide. Lors du dépôt, il remplit les vias 201 de la couche 101 sous-jacente (figure 11f).

Dans une première variante, l'étape d) peut être réalisée en :

-   formant un film plein 102' constitué du matériau du deuxième film 102 (figure 11F),
-   gravant le film plein 102', de manière à former au moins une cavité et à rendre accessible l'au moins une piste électriquement conductrice 200 (figure 11H),
-   reportant un composant électronique 300 respectif dans chaque cavité et en connectant chaque composant électronique 300 avec l'une au moins piste électriquement conductrice 200, chaque connexion étant réalisée par l'intermédiaire d'un élément de connexion 400 respectif (figure 11I).

Les cavités dans le deuxième film 102 peuvent être gravées par des procédés de microélectronique connus de l'homme du métier: gravure plasma, gravure laser, gravure ionique, gravure chimique.

Après l'étape d), et préalablement à l'étape de gravure, une couche de compensation 500 peut être formée sur le deuxième film 102 (figure 11G).

Selon une autre variante, l'étape d) peut être réalisée en :

- reportant et connectant chaque composant électronique 300 chacun sur l'une piste électriquement conductrice 200,
- formant le deuxième film 102 sur l'au moins un composant électronique 300 et sur le premier film 101.

Dans cette variante, lorsque le deuxième film 102 est suffisamment épais pour encapsuler complètement les composants 300, à la fois latéralement et sur le dessus, il n'y aura pas besoin de déposer un troisième film 103.

Les composants électroniques 300 sont reportés, face active vers le bas (en « flip chip »), c'est-à-dire la face comportant les plots d'interconnexion en regard du film 101. Le report sera réalisé par des procédés de microélectronique connus de l'homme du métier. Le deuxième film 102 entoure les composants 300 en pouvant laisser une tranchée 112 autour d'eux. La tranchée 112 est alors une tranchée dite de scellement. Elle sera remplie par le matériau de la couche supérieure lors de sa formation (étape e).

L'interconnexion électrique et mécanique des composants sur les pistes électriques pourra être réalisée à l'aide de billes fusibles (par exemple en SnAg, SnPb ou SnAgCu etc.) ou bien à l'aide d'une colle conductrice. Elle peut également être réalisée par un bossage en or plus connu sous le nom de « stud bump » ou « accu bump ». L'élément d'interconnexion 400 est formé lors de cette étape.

Il pourra être envisagé de réaliser une finition spécifique (par exemple en un multi-couche Ti\Ni\Au connu sous le nom de UBM (« Under Bump Metallization »)) sur les pistes électriques et/ou sous les plots des composants électroniques, pour favoriser la liaison.

Une colle non conductrice pourra être ajoutée sous les composants 300 de façon à renforcer la cohésion mécanique.

Dans une variante, on pourra connecter les composants 300 à leur piste électrique 200 par un assemblage mécanique sans fusion de type micro-insert ou micro-tube connu de l'homme du métier. Il s'agit, par exemple, de faire croître des micro-piliers ou des micro-tubes métalliques, ayant un diamètre et/ou une hauteur de quelques $\mu$m à quelques dizaines de $\mu$m, sur les plots électriques des composants 300. Lors du report du composant 300, une pression suffisante est exercée pour que ces protubérances s'insèrent mécaniquement dans la couche métallique d'accueil.

Une étape de thermocompression peut être réalisée pour favoriser l'interconnexion électrique.

Dans un mode de réalisation particulier, les composants 300 peuvent être amincis (figure 11J). L'amincissement des composants 300 est réalisé après report des composants 300 et avant le dépôt du film qui va les recouvrir (film 102 ou film 103 selon le mode de réalisation). Il est réalisé par tout procédé de microélectronique connu de l'homme du métier, par exemple par meulage et/ou polissage, ou encore par planarisation mécano-chimique (ou CMP pour « Chemical mechanical polishing/planarization »). Les composants 300 classiquement utilisés dans l'industrie microélectronique ont des épaisseurs allant de 200 $\mu$m à 700 $\mu$m. Ces composants pourront être amincis jusqu'à quelques microns d'épaisseur, de préférence de 5 $\mu$m à 350 $\mu$m, et encore plus préférentiellement de 20 $\mu$m à 70 $\mu$m.

Le troisième film 103 est ensuite déposé sur le deuxième film 102 et sur les composants 300 de manière à les recouvrir (étape e, figure 11K). Le troisième film 103 flexible est, de préférence, déposé à l'état liquide. Lors du dépôt, il remplit les tranchées de scellement 112 de la couche sous-jacente.

Les différents films 101, 102, 103, lorsqu'ils sont en polymère, sont avantageusement formés à une température compatible avec une évaporation rapide et contrôlée du solvant dans lequel se trouve le polymère.

Selon une variante du procédé non représentée, après l'étape e), une couche de compensation 500 peut être formée sur le troisième film 103.

En variante, on dépose sur le deuxième film 102 une couche épaisse de verre (environ 100 $\mu$m d'épaisseur), que l'on grave localement sur une partie seulement de son épaisseur. On réalise ainsi une couche de verre comportant des surépaisseurs qui forment les portions discrètes de la couche de compensation 500. En d'autres termes, la couche de verre après gravure forme un troisième film en verre 103 et une couche de compensation 500 superposés.

La structure 100 est ensuite séparée du substrat 700 (étape f, figure 11L).

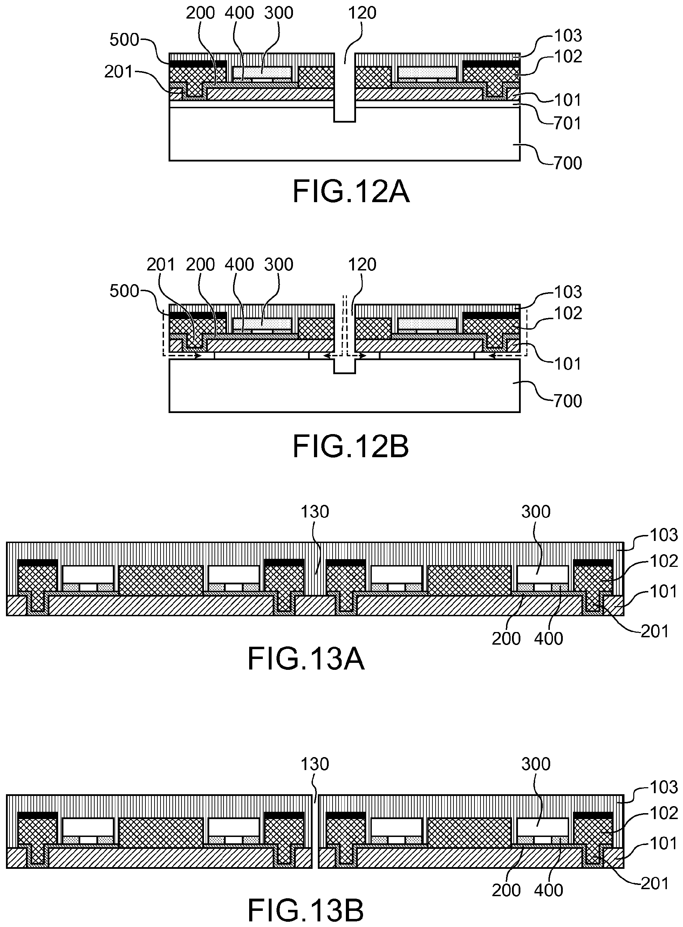

Le retrait du substrat 700 sera, avantageusement, réalisé grâce à la couche sacrificielle 701. La couche sacrificielle 701 peut être gravée pour séparer le substrat 700 de la structure électronique souple 100. Elle peut être gravée chimiquement, par exemple avec de l'acide fluorhydrique. Elle peut être attaquée latéralement. Comme représenté sur les figures 12A et 12B, avant l'étape f), un ou plusieurs trous traversant 120 la structure 100, peuvent être réalisés de manière à rendre la couche sacrificielle 701 accessible en différents endroits, et non pas seulement au niveau des bords du substrat 700, pour faciliter l'étape de gravure.

Selon une variante, la couche sacrificielle 701 peut être retirée mécaniquement, par exemple, par meulage et/ou polissage. La couche sacrificielle 701 peut, avantageusement, servir de couche d'arrêt.

Selon un autre mode de réalisation, représenté sur les figures 13A et 13B, lorsque la structure 100 comprend plusieurs composants disposés dans une même couche, celle-ci peut être découpée en plusieurs parties, avant ou après le retrait du substrat 700. Avantageusement, la zone de découpe 130, une fois découpée, lors du retrait du substrat 700, peut servir comme zone d'infiltration (trou traversant 120) pour la solution de gravure, pour faciliter la gravure de la couche sacrificielle 701.

De manière avantageuse, au niveau de la zone de découpe 130, le deuxième film 102 est gravé, de manière à former localement une cavité qui est remplie par le troisième film 103 lors de sa formation. Cela peut présenter un intérêt lorsque l'on veut encapsuler complètement les composants électroniques 300 dans un matériau ayant des propriétés spécifiques, comme par exemple une biocompatibilité.

**Revendications**

1. Structure électronique souple (100) comprenant :

   - un premier film (101), en un premier polymère ou en verre,
   - un deuxième film (102), en un deuxième polymère, dans lequel est disposé au moins un composant électronique (300), le deuxième film (102) recouvrant le premier film (101),
   - au moins une piste électriquement conductrice (200), disposée entre le premier film (101) et le deuxième film (102), et électriquement connectée(s) chacune à l'un parmi l'au moins un composant électronique (300), par un élément d'interconnexion (400) respectif,
   chaque élément d'interconnexion (400) étant disposé à proximité du plan neutre de la structure (100), c'est-à-dire passant par un plan dont la distance au plan neutre est inférieure ou égale à 20% de l'épaisseur totale de la structure,
   la structure électronique souple (100) comprenant en outre au moins une couche de compensation (500) discontinue configurée pour positionner le plan neutre à un emplacement voulu, au plus près de chaque élément d'interconnexion, la couche de compensation (500) discontinue étant formée d'une ou plusieurs portions discrètes qui s'étendent chacune en vis-à-vis de la piste électriquement conductrice (200) ou en vis-à-vis du composant électronique (300) ;
   et la structure électronique souple (100) étant **caractérisée en ce qu'**un via (201) métallisé respectif, électriquement connecté à la piste électriquement conductrice (200), s'étend dans le premier film (101) de manière à connecter le composant électronique (300) à travers le premier film (101), le via (201) métallisé étant rempli par le deuxième polymère.

2. Structure (100) selon la revendication 1, **caractérisée en ce qu'**elle comporte en outre un troisième film (103) en un troisième polymère ou en verre, recouvrant le deuxième film (102).

3. Structure (100) selon la revendication 2, **caractérisée en ce que** le troisième film (103) et la couche de compensation (500) sont en verre, et formés ensemble d'un seul tenant.

4. Structure (100) selon l'une quelconque des revendications 1 à 3, **caractérisée en ce que** le deuxième film (102) comprend plusieurs composants électroniques (300) de différentes surfaces, et **en ce que** la couche de compensation (500) recouvre le composant électronique (300) de plus petite surface pour le rigidifier.

5. Structure (100) selon l'une des revendications 1 à 4, **caractérisée en ce que** l'au moins une couche de compensation (500) est en un même matériau que l'au moins une piste électriquement conductrice (200).

6. Structure (100) selon l'une quelconque des revendications 1 à 5, **caractérisée en ce qu'**elle comporte en outre au moins une pointe (202) métallisée, électriquement connectée à la piste électriquement conductrice (200) par l'intermédiaire du via (201) correspondant, et faisant saillie à partir du premier film (101) du côté opposé à la piste électriquement conductrice (200), la pointe étant remplie par le deuxième polymère.

7. Structure (100) selon l'une des revendications 2 à 6, **caractérisée en ce qu'**elle comprend au moins une tranchée de scellement (112), chacune entourant l'un parmi l'au moins un composant électronique (300), le polymère du troisième film (103) remplissant la tranchée de scellement (112).

8. Structure (100) selon l'une quelconque des revendications 2 à 7, **caractérisée en ce qu'**au moins un trou traversant (600) s'étend dans le premier film (101) et/ou dans le troisième film (103) chacun de manière à rendre accessible l'un parmi l'au moins un composant électronique (300).

9. Procédé de préparation d'une structure électronique souple (100) telle que définie dans l'une quelconque des revendications précédentes, ledit procédé comprenant les étapes successives suivantes :

a) fourniture d'un substrat (700),
b) formation du premier film (101) en un premier polymère ou en verre, sur le substrat (700),
c) formation de l'au moins une piste électriquement conductrice (200) sur le premier film (101),
d) formation du deuxième film (102) en un deuxième polymère, dans lequel est disposé au moins un composant électronique (300), chaque composant électronique (300) étant électriquement connecté avec la piste électriquement conductrice (200) par un élément d'interconnexion (400) respectif,
e) séparation du substrat (700) de la structure électronique souple (100),

le procédé comprenant en outre au moins une étape de formation d'au moins une couche de compensation (500) configurée pour positionner le plan neutre à un emplacement voulu, au plus près de chaque élément d'interconnexion.

10. Procédé de préparation selon la revendication 9, **caractérisé en ce que** l'étape d) comprend les sous étapes suivantes :

- formation d'un film plein constitué du matériau du deuxième film (102),
- gravure du film plein, de manière à former au moins une cavité, chaque cavité rendant accessible la piste électriquement conductrice (200),

report d'un composant électronique (300) respectif dans les cavités et connexion de chaque composant électronique (300) avec au moins la piste électriquement conductrice (200), chaque connexion étant réalisée par l'intermédiaire d'un élément d'interconnexion (400) respectif.

11. Procédé de préparation selon la revendication 9, **caractérisé en ce que** l'étape d) comprend les sous étapes suivantes :

- report et connexion d'au moins un composant électronique (300), chacun avec au moins la piste électriquement conductrice (200), chaque connexion étant réalisée par l'intermédiaire d'un élément d'interconnexion (400) respectif,
- formation du deuxième film (102) sur l'au moins un composant électronique (300) et sur le premier film (101).

12. Procédé selon l'une des revendications 9 à 11, **caractérisé en ce que**, entre l'étape a) et l'étape b), une couche sacrificielle (701) est déposée sur le support (700) et **en ce que** lors de l'étape e), la couche sacrificielle (701) est gravée pour séparer le support (700) de la structure électronique souple (100).

13. Procédé selon l'une quelconque des revendications 9 à 12, adapté pour réaliser une structure électronique souple (100) comportant en outre un troisième film (103) en un troisième polymère ou en verre, recouvrant le deuxième film (102), le procédé étant **caractérisé en ce que** :

- avant l'étape b), l'une parmi l'au moins une couche de compensation (500) est formée sur le substrat (700), et/ou
- après l'étape d), l'une parmi l'au moins une couche de compensation (500) est formée sur le deuxième film (102), et/ou
- après l'étape d), l'une parmi l'au moins une couche de compensation (102) est formée sur le troisième film (103).

14. Procédé selon l'une quelconque des revendications 9 à 13, **caractérisé en ce que** le premier polymère est un polyimide, un polysiloxane, un parylène ou un polymère thermoplastique.

15. Procédé selon l'une quelconque des revendications 9 à 14, **caractérisé en ce que** le substrat (700) comporte au moins un renfoncement (702) en forme de pointe, formant une empreinte pour former l'au moins une pointe (202) métallisée faisant saillie à partir du premier film (101), du côté opposé à l'au moins une piste électriquement conductrice (200).

**Patentansprüche**

1. Flexible elektronische Struktur (100), umfassend:

- eine erste Folie (101), aus einem ersten Polymer oder Glas,
- eine zweite Folie (102) aus einem zweiten Polymer, in der mindestens eine elektronische Komponente (300) angeordnet ist, wobei die zweite Folie (102) die erste Folie (101) abdeckt,
- mindestens eine elektrisch leitende Bahn (200), die zwischen der ersten Folie (101) und der zweiten Folie (102) angeordnet und jeweils mit einer der mindestens einen elektronischen Komponente (300) durch ein jeweiliges Verbindungselement (400) elektrisch verbunden ist, wobei jedes Verbindungselement (400) in der Nähe der neutralen Ebene der Struktur (100) angeordnet ist, d. h. durch eine Ebene verläuft, deren Abstand zu der neutralen Ebene kleiner oder gleich 20 % der Gesamtdicke der Struktur ist, wobei die flexible elektronische Struktur (100) ferner mindestens eine diskontinuierliche Kompensationsschicht (500) umfasst, die so konfiguriert ist, dass sie die neutrale Ebene an einer gewünschten Stelle so nah wie möglich an jedem Verbindungselement positioniert, wobei die diskontinuierliche Kompensationsschicht (500) aus einem oder mehreren diskreten Abschnitten gebildet ist, die sich jeweils gegenüber der elektrisch leitenden Bahn (200) oder gegenüber der elektronischen Komponente (300) erstrecken;

und wobei die flexible elektronische Struktur (100) **dadurch gekennzeichnet ist, dass** sich ein jeweiliges metallisiertes Via (201), das elektrisch mit der elektrisch leitenden Bahn (200) verbunden ist, in die erste Folie (101) erstreckt, um die elektronische Komponente (300) durch die erste Folie (101) zu verbinden, wobei das metallisierte Via (201) mit dem zweiten Polymer gefüllt ist.

2. Struktur (100) nach Anspruch 1, **dadurch gekennzeichnet, dass** sie ferner eine dritte Folie (103) aus einem dritten Polymer oder Glas aufweist, die die zweite Folie (102) abdeckt.

3. Struktur (100) nach Anspruch 2, **dadurch gekennzeichnet, dass** die dritte Folie (103) und die Kompensationsschicht (500) aus Glas bestehen und zusammen aus einem Stück gebildet sind.

4. Struktur (100) nach einem der Ansprüche 1 bis 3, **dadurch gekennzeichnet, dass** die zweite Folie (102) mehrere elektronische Komponenten (300) mit unterschiedlichen Oberflächen umfasst und dass die Kompensationsschicht (500) die elektronische Komponente (300) mit kleinerer Oberfläche zur Versteifung abdeckt.

5. Struktur (100) nach einem der Ansprüche 1 bis 4, **dadurch gekennzeichnet, dass** die mindestens eine Kompensationsschicht (500) aus demselben Material wie die mindestens eine elektrisch leitende Bahn (200) besteht.

6. Struktur (100) nach einem der Ansprüche 1 bis 5, **dadurch gekennzeichnet, dass** sie ferner mindestens eine metallisierte Spitze (202) aufweist, die über das entsprechende Via (201) elektrisch mit der elektrisch leitenden Bahn (200) verbunden ist und aus der ersten Folie (101) auf der der elektrisch leitenden Bahn (200) gegenüberliegenden Seite herausragt, wobei die Spitze mit dem zweiten Polymer gefüllt ist.

7. Struktur (100) nach einem der Ansprüche 2 bis 6, **dadurch gekennzeichnet, dass** sie mindestens einen Versiegelungsgraben (112) umfasst, der jeweils eine der mindestens einen elektronischen Komponente (300) umgibt, wobei das Polymer der dritten Folie (103) den Versiegelungsgraben (112) füllt.

8. Struktur (100) nach einem der Ansprüche 2 bis 7, **dadurch gekennzeichnet, dass** sich jeweils mindestens ein Durchgangsloch (600) in der ersten Folie (101) und/oder in der dritten Folie (103) erstreckt, um eine der mindestens einen elektronischen Komponente (300) zugänglich zu machen.

9. Vorbereitungsverfahren einer flexiblen elektronischen Struktur (100) gemäß einem der vorhergehenden Ansprüche, wobei das Verfahren die folgenden aufeinanderfolgenden Schritte umfasst:

a) Bereitstellung eines Substrats (700),
b) Bildung der ersten Folie (101) aus einem ersten Polymer oder Glas auf dem Substrat (700),
c) Bildung der mindestens einen elektrisch leitenden Bahn (200) auf der ersten Folie (101),
d) Bildung der zweiten Folie (102) aus einem zweiten Polymer, in dem mindestens eine elektronische Komponente (300) angeordnet ist, wobei jede elektronische Komponente (300) durch ein jeweiliges Verbindungselement (400) elektrisch mit der elektrisch leitenden Bahn (200) verbunden ist,
e) Trennung des Substrats (700) von der flexiblen elektronischen Struktur (100),

wobei das Verfahren ferner mindestens einen Schritt der Bildung mindestens einer Kompensationsschicht (500) umfasst, die so konfiguriert ist, dass sie die neutrale Ebene an einer gewünschten Stelle, möglichst nahe an jedem Verbindungselement, positioniert.

10. Vorbereitungsverfahren nach Anspruch 9, **dadurch gekennzeichnet, dass** der Schritt d) folgende Unterschritte umfasst:

- Bildung einer Vollfolie, die aus dem Material der zweiten Folie (102) besteht,
- Ätzung der Vollfolie, sodass mindestens ein Hohlraum gebildet wird, wobei jeder Hohlraum die elektrisch leitende Bahn (200) zugänglich macht,

Übertragung einer jeweiligen elektronischen Komponente (300) in die Hohlräume und Verbindung jeder elektronischen Komponente (300) mit mindestens der elektrisch leitenden Bahn (200), wobei jede Verbindung über ein jeweiliges Verbindungselement (400) hergestellt wird.

11. Vorbereitungsverfahren nach Anspruch 9, **dadurch gekennzeichnet, dass** der Schritt d) folgende Unterschritte umfasst:

- Übertragung und Verbindung mindestens einer elektronischen Komponente (300), jeweils mit mindestens der elektrisch leitenden Bahn (200), wobei jede Verbindung über ein jeweiliges Verbindungselement (400) hergestellt wird,
- Bildung der zweiten Folie (102) auf der mindestens einen elektronischen Komponente (300) und auf der ersten Folie (101).

12. Verfahren nach einem der Ansprüche 9 bis 11, **dadurch gekennzeichnet, dass** zwischen Schritt a) und Schritt b) eine Opferschicht (701) auf den Träger (700) aufgebracht wird und dass bei Schritt e) die Opferschicht (701) graviert wird, um den Träger (700) von der flexiblen elektronischen Struktur (100) zu trennen.

13. Verfahren nach einem der Ansprüche 9 bis 12, geeignet, um eine flexible elektronische Struktur (100) herzustellen, die ferner eine dritte Folie (103) aus einem dritten Polymer oder Glas aufweist, die die zweite Folie (102) abdeckt, wobei das Verfahren **dadurch gekennzeichnet ist, dass**:

- vor Schritt b) eine der mindestens einen Kompensationsschicht (500) auf dem Substrat (700) ausgebildet ist, und/oder
- nach Schritt d) eine der mindestens einen Kompensationsschicht (500) auf der zweiten Folie (102) ausgebildet ist, und/oder
- nach Schritt d) eine der mindestens einen Kompensationsschicht (102) auf der dritten Folie (103) ausgebildet ist.

14. Verfahren nach einem der Ansprüche 9 bis 13, **dadurch gekennzeichnet, dass** das erste Polymer ein Polyimid, ein Polysiloxan, ein Parylen oder ein thermoplastisches Polymer ist.

15. Verfahren nach einem der Ansprüche 9 bis 14, **dadurch gekennzeichnet, dass** das Substrat (700) mindestens eine spitzenförmige Vertiefung (702) aufweist, die einen Abdruck bildet, um die mindestens eine metallisierte Spitze (202) zu bilden, die aus der ersten Folie (101) herausragt, auf der der mindestens einen elektrisch leitenden Bahn (200) gegenüberliegenden Seite.

**Claims**

1. A flexible electronic structure (100) comprising:

- a first film (101), made of a first polymer or of glass,
- a second film (102), made of a second polymer, wherein at least one electronic component (300) is disposed, the second film (102) covering the first film (101),
- at least one electrically conductive track (200), disposed between the first film (101) and the second film (102), and each electrically connected to one of the at least one electronic component (300), by a respective interconnection element (400),
each interconnection element (400) being disposed near the neutral plane of the structure (100), that is to say passing through a plane whose distance from the neutral plane is less than or equal to 20% of the total thickness of the structure,
the flexible electronic structure (100) further comprising at least one discontinuous compensation layer (500), configured to position the neutral plane at a desired location, as close as possible to each interconnection element, the discontinuous compensation layer (500) being formed of one or more discrete portions which each extends opposite the electrically conductive track (200) or opposite the electronic component (300);
and the flexible electronic structure (100) being **characterized in that** a respective metal via (201), electrically connected to the electrically conductive track (200), extends in the first film (101) so as to connect the electronic component (300) through the first film (101), the metal via (201) being filled with the second polymer.

2. The structure (100) according to claim 1, **characterized in that** it further includes a third film (103) made of a third polymer or of glass, covering the second film (102).

3. The structure (100) according to claim 2, **characterized in that** the third film (103) and the compensation layer (500) are made of glass, and formed together integrally.

4. The structure (100) according to any one of claims 1 to 3, **characterized in that** the second film (102) comprises several electronic components (300) of different surface areas, and **in that** the compensation layer (500) covers the electronic component (300) of smaller surface area so as to stiffen it.

5. The structure (100) according to any one of claims 1 to 4, **characterized in that** the at least one compensation layer (500) is made of the same material as at least one electrically conductive track (200).

6. The structure (100) according to any one of claims 1 to 5, **characterized in that** it further includes at least one metal tip (202), electrically connected to the electrically conductive track (200) by means of the corresponding via (201), and protruding from the first film (101) on the side opposite to the electrically conductive track (200), the tip being filled with the second polymer.

7. The structure (100) according to one of claims 2 to 6, **characterized in that** it comprises at least one sealing trench (112), each surrounding one of the at least one electronic component (300), the polymer of the third film (103) filling the sealing trench (112).

8. The structure (100) according to any one of claims 2 to 7, **characterized in that** at least one through-hole (600) extends in the first film (101) and/or in the third film (103) each so as to make one of the at least one electronic component (300) accessible.

9. A method for producing a flexible electronic structure (100) as defined in any one of the preceding claims, said method comprising the following successive steps:

    a) providing a substrate (700),
    b) forming the first film (101) made of a first polymer or of glass, on the substrate (700),
    c) forming at least one electrically conductive track (200) on the first film (101),
    d) forming the second film (102) made of a second polymer, wherein at least one electronic component (300) is disposed, each electronic component (300) being electrically connected with the electrically conductive track (200) by a respective interconnection element (400),
    e) separating the substrate (700) from the flexible electronic structure (100),

the method further comprising at least one step of forming at least one compensation layer (500) configured to position the neutral plane at a desired location, as close as possible to each interconnection element.

10. The preparation method according to claim 9, **characterized in that** step d) comprises the following sub-steps:

    - forming a solid film made of the material of the second film (102),
    - etching the solid film, so as to form at least one cavity, each cavity making the electrically conductive track (200) accessible,
    - transferring a respective electronic component (300) into the cavities and connecting each electronic component (300) with at least the electrically conductive track (200), each connection being made by means of a respective interconnection element (400).

11. The preparation method according to claim 9, **characterized in that** step d) comprises the following sub-steps:

    - transferring and connecting at least one electronic component (300), each with at least the electrically conductive track (200), each connection being made by means of a respective interconnection element (400),
    - forming the second film (102) on the at least one electronic component (300) and on the first film (101).

12. The method according to one of claims 9 to 11, **characterized in that**, between step a) and step b), a sacrificial layer (701) is deposited on the support (700) and **in that** during step e), the sacrificial layer (701) is etched to separate the support (700) from the flexible electronic structure (100).

13. The method according to any one of claims 9 to 12, adapted to produce a flexible electronic structure (100) further including a third film (103) made of a third polymer or of glass, covering the second film (102), the method being **characterized in that**:

    - before step b), one of the at least one compensation layer (500) is formed on the substrate (700), and/or
    - after step d), one of the at least one compensation layer (500) is formed on the second film (102), and/or
    - after step d), one of the at least one compensation layer (102) is formed on the third film (103).

14. The method according to any one of claims 9 to 13, **characterized in that** the first polymer is a polyimide, a polysiloxane, a parylene or a thermoplastic polymer.

15. The method according to any one of claims 9 to 14, **characterized in that** the substrate (700) includes at least one recess (702) in the shape of a tip, forming an indentation to form the at least one metal tip (202) protruding from the first film (101), on the side opposite to the at least one electrically conductive track (200).

FIG.1A

FIG.1B

FIG.2

FIG.3

FIG.4

FIG.5A

FIG.5B

FIG.6

FIG.7

FIG.8

FIG.9

FIG.10

FIG.11A

FIG.11B

FIG.11C

FIG.11D

FIG.11E

FIG.11F

FIG.11G

FIG.11H

FIG.11I

FIG.11J

FIG.11K

FIG.11L

FIG.11M

FIG.12A

FIG.12B

FIG.13A

FIG.13B

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20070134849 A
- US 20060097373 A
- US 2018042107 A
- WO 2013082537 A

**Littérature non-brevet citée dans la description**

- **SUO et al.** Interface crack between two elastic layers. *International Journal of Fracture*, 1990, vol. 43, 1-18
- **EBERL et al.** Mechanical Characterization of Coatings Using Microbeam Bending and Digital Image Correlation Techniques. *Experimental Mechanics*